# EUROPEAN PATENT SPECIFICATION

(11) **EP 1 143 806 B1**
(45) Date of publication and mention of the grant of the patent: **19.04.2006**
(21) Application number: 00979476.9
(22) Date of filing: 21.09.2000
(51) Int. Cl.: A23K 1/16, A23K 1/18

(54) **METHOD FOR INCREASING PET ACTIVITY**
VERFAHREN ZUR STEIGERUNG DER AKTIVITÄT VON HAUSTIEREN
PROCEDE PERMETTANT D'AUGMENTER L'ACTIVITE CHEZ LES ANIMAUX DOMESTIQUES

(30) Priority: 22.09.1999 US 155451 P
(43) Date of publication of application: 17.10.2001
(73) Proprietor: SOCIETE DES PRODUITS NESTLE S.A., 1800 Vevey (CH)
(72) Inventor: SCHIFFRIN, Eduardo, CH-1023 Crissier (CH); CZARNECKI, Gail, Easton, MO 64443 (US)
(74) Representative: Scott, Fiona Penelope Elaine
(86) International application number: PCT/EP2000/009444
(87) International publication number: WO 2001/021008

(56) References cited:
- EP-A- 0 042 303
- EP-A- 0 630 576
- EP-A- 0 850 569
- EP-A- 0 862 863
- WO-A-99/22604
- FR-A- 2 594 644
- US-A- 5 776 524
- US-A- 6 156 355
- PATENT ABSTRACTS OF JAPAN vol. 2000, no. 08, 6 October 2000 (2000-10-06) & JP 2000 125778 A (BIO TEC JAPAN:KK;YUKIGUNI MAITAKE CO LTD; KOUKIN TECHNO:KK), 9 May 2000 (2000-05-09)
- LOWE J A: "CANINE NUTRITION - RECENT ADVANCES" 1988 , CONFERENCE ON BIOTECHNOLOGY IN THE FEED INDUSTRY. PROCEEDINGS OF ALLTECH XP000670866 page 285, paragraph 3

## Description

### Field of the invention

This invention relates to a method of increasing the activity of pets; especially elderly cats and dogs.

### Background of the invention

Once pets reach an age where their systems start to slow down, certain symptoms of aging begin to manifest themselves; joint stiffness, energy loss, weight gain, increased water intake, digestive system problems, a dull, dry coat and flaky skin. For dogs, this usually starts becoming noticeable at about 5 years for larger breeds and about 7 years for smaller breeds. For cats, this usually starts becoming noticeable at about 7 years. However, the process is different for every animal and there is no standard age at which the symptoms become manifest.

The onset of many of these symptoms may be delayed by feeding the animal a complete, well-balanced diet over its life. Further, the condition of the elderly animal can be improved through nutrition. In particular, healthy animals should be fed a balanced, maintenance food that contains high quality protein, lower amounts of fat to reduce energy intake, dietary fiber, and antioxidants. Also, regular exercise is important to maintain muscle tone, enhance circulation, promote digestion and prevent weight gain.

However, despite good nutrition and regular exercise, many older animals are lethargic and appear to lack energy. Similar problems may also occur in younger animals.

Therefore there remains a need for ways of improving the activity of pets; especially older pets.

### Summary of the invention

The invention is defined in the claims.

Accordingly, in one aspect, this invention provides a method for improving activity in a pet, the method comprising administering to the pet a nutritional agent which promotes the growth of bifido- and lactic-bacteria in the gastro-intestinal tract of the pet.

It has been surprisingly discovered that administering to a pet a nutritional agent which promotes the growth of bifido- and lactic-bacteria in the gastro-intestinal tract of the pet results in improved activity levels in the pet. This is particularly noticeable in elderly pets. Without wishing to be bound by theory, it is believed that, amongst other mechanisms, increasing the concentrations of bifido- and lactic-bacteria in the gastro-intestinal tract of the pet produces nutrients and/or increases the absorption of nutrients which provides the pet with better nutrition and more energy. Further, increasing the concentrations of bifido- and lactic-bacteria in the gastro-intestinal tract of the pet reduces the concentrations of pathogenic bacteria in the gastro-intestinal tract and this may improve systemic inflammatory status; leading to less joint stiffness.

In another aspect, this invention provides a method for ameliorating joint stiffness in a pet, the method comprising administering to the pet a nutritional agent which promotes the growth of bifido- and lactic-bacteria in the gastro-intestinal tract of the pet. The nutritional agent preferably assists in improving - by reducing - the systemic inflammatory status in the pet. This may be achieved by reducing concentrations of pathogenic bacteria known to contribute to an increased inflammatory status.

The pet is preferably an elderly pet; especially an elderly dog. The dog may be older than 5 years of age; for example older than 7 years of age. The cat may be older than 7 years of age.

Preferably the nutritional agent is administered to the pet in the form of a complete and nutritionally balanced pet food.

The nutritional agent may be a prebiotic, a probiotic micro-organism, or a fermentation product obtained from the fermentation of probiotic micro-organisms.

In this specification:-
"Prebiotic" means a substance or compound which is fermented by the intestinal flora of the pet and hence promotes the growth or development of bifido- and lactic-bacteria in the gastro-intestinal tract of the pet at the expense of pathogenic bacteria. The result of this fermentation is a release of fatty acids, in particular short-chain fatty acids in the colon. This has the effect of reducing the pH value in the colon.
"Probiotic micro-organism" means a micro-organism which beneficially affects a host by improving its intestinal microbial balance (Fuller, R; 1989; J. Applied Bacteriology, 66: 365-378). In general, probiotic micro-organisms produce organic acids such as lactic acid and acetic acid which inhibit the growth of pathogenic bacteria such as *Clostridium perfringens* and *Helicobacter pylori.*

### Detailed description of preferred embodiments of the invention.

This invention is based upon the discovery that the activity levels in a pet may be improved by administering to the pet a nutritional agent which promotes the growth of bifido- and lactic-bacteria in the gastro-intestinal tract of the pet. The activity levels of elderly pets, such as elderly dogs, is particularly improved.

The nutritional agent may be a prebiotic, a probiotic micro-organism, or a fermentation product obtained from fermentation of a probiotic micro-organism. Further, combinations of the prebiotics, probiotic micro-organisms and fermentation products may be administered to the pet.

Suitable prebiotics include oligosaccharides, such as inulin and its hydrolysis products commonly known as fructooligosaccharides, galacto-oligosaccarides, xylo-oligosaccharides or oligo derivatives of starch. Combinations of starches and gums may also be used.

The prebiotics may be provided in any suitable form. For example, the prebiotic may be provided in the form of plant material which contains the prebiotic. Suitable plant materials includes asparagus, artichokes, onions, wheat, yacon or chicory, or residues of these plant materials. Alternatively, the prebiotic may be provided as an inulin extract. Extracts from chicory are particularly suitable. Suitable inulin extracts may be obtained from Orafti SA of Tirlemont 3300, Belgium under the trade mark "Raftiline". For example, the inulin may be provided in the form of Raftiline®ST which is a fine white powder which contains about 90 to about 94% by weight of inulin, up to about 4% by weight of glucose and fructose, and about 4 to 9% by weight of sucrose. Alternatively, the prebiotic may be in the form of a fructooligosaccharide such as obtained from Orafti SA of Tirlemont 3300, Belgium under the trade mark "Raftilose". For example, the inulin may be provided in the form of Raftilose®P95. Otherwise, the fructooligosaccharides may be obtained by hydrolyzing inulin, by enzymatic methods, or by using micro-organisms.

The probiotic micro-organism may be selected from one or more micro-organisms suitable for animal consumption and which is able to improve the microbial balance in the intestine.

Examples of suitable probiotic micro-organisms include yeasts such as *Saccharomyces, Debaromyces, Candida, Pichia* and *Torulopsis,* moulds such as *Aspergillus, Rhizopus, Mucor,* and *Penicillium* and *Torulopsis* and bacteria such as the genera *Bifidobacterium, Bacteroides, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus* and *Lactobacillus.* Specific examples of suitable probiotic micro-organisms are: *Saccharomyces cereviseae, Bacillus coagulans, Bacillus licheniformis, Bacillus subtilis, Bifidobacterium bifidum, Bifidobacterium infantis, Bifidobacterium longum, Enterococcus faecium, Enterococcus faecalis, Lactobacillus acidophilus, Lactobacillus alimerrtarius, Lactobacillus casei* subsp. *casei, Lactobacillus casei Shirota, Lactobacillus curvatus, Lactobacillus delbruckii* subsp. *lactis, Lactobacillus farciminus, Lactobacillus gasseri, Lactobacillus helveticus, Lactobacillus johnsonii, Lactobacillus reuteri, Lactobacillus rhamnosus (Lactobacillus* GG), *Lactobacillus sake, Lactococcus lactis, Micrococcus varians, Pediococcus acidilactici, Pediococcus pentosaceus, Pediococcus acidilactici, Pediococcus halophilus, Streptococcus faecalis, Streptococcus thermophilus, Staphylococcus carnosus,* and *Staphylococcus xylosus.* The probiotic micro-organisms may be in powdered, dried form; especially in spore form for micro-organisms which form spores. Further, if desired, the probiotic micro-organism may be encapsulated to further increase the probability of survival; for example in a sugar matrix, fat matrix or polysaccharide matrix.

The nutritional agent may be administered to the pet as a supplement to the pet's normal diet or as a component of a nutritionally complete pet food. It is preferred to include the nutritional agent in a nutritionally complete pet food.

The nutritionally complete pet food may be in any suitable form; for example in dried form, semi-moist form and wet form. These pet foods may be produced as is conventional. Apart from the nutritional agent, these pet foods may include any one or more of a starch source, a protein source and lipid source. Suitable starch sources are, for example, grains and legumes such as corn, rice, wheat, barley, oats, soy, and mixtures of these. Suitable protein sources may be selected from any suitable animal or vegetable protein source; for example meat and meal, poultry meal, fish meal, soy protein concentrates, milk proteins, gluten, and the like. For elderly animals, it is preferred for the protein source to contain a high quality protein. Suitable lipid sources include meats, animal fats and vegetable fats. The choice of the starch, protein and lipid sources will be largely determined by the nutritional needs of the animal, palatability considerations, and the type of product produced. Further, various other ingredients, for example, sugar, salt, spices, seasonings, vitamins, minerals, flavoring agents, fats and the like may also be incorporated into the pet food as desired.

For elderly pets, the pet food preferably contains proportionally less fat than pet foods for younger pets. Further, the starch sources may include one or more of rice, barley, wheat and corn.

For dried pet foods a suitable process is extrusion cooking, although baking and other suitable processes may be used. When extrusion cooked, the dried pet food is usually provided in the form of a kibble. If a prebiotic is used, the prebiotic may be admixed with the other ingredients of the dried pet food prior to processing. A suitable process is described in European patent application No 0850569. If a probiotic micro-organism is used, the organism is best coated onto or filled into the dried pet food. A suitable process is described in European patent application No 0862863.

For wet foods, the processes described in US patents 4,781,939 and 5,132,137 may be used to produce simulated meat products. Other procedures for producing chunk type products may also be used; for example cooking in a steam oven. Alternatively, loaf type products may be produced by emulsifying a suitable meat material to produce a meat emulsion, adding a suitable gelling agent, and heating the meat emulsion prior to filling into cans or other containers.

The maximum level of prebiotic in the pet food is preferably about 20% by weight; especially about 10% by weight. However, considerably lower levels are found to be effective in increasing activity levels. For example, the prebiotic may comprise about 0.1 % to about 5% by weight of the pet food. For pet foods which use chicory as the prebiotic, the chicory may be included to comprise about 0.5% to about 10% by weight of the feed mixture; more preferably about 1% to about 5% by weight.

If a probiotic micro-organism is used, the pet food preferably contains about 10⁴to about 10¹⁰ cells of the probiotic micro-organism per gram of the pet food; more preferably about 10⁶ to about 10⁸ cells of the probiotic micro-organism per gram. The pet food may contain about 0.25% to about 20% by weight of the mixture of the probiotic micro-organism; preferably about 0.5% to about 6% by weight; for example about 3% to about 6% by weight.

The pet foods may contain other active agents such as long chain fatty acids. Suitable long chain fatty acids include alpha-linoleic acid, gamma linolenic acid, linoleic acid, eicosapentanoic acid, and docosahexanoic acid. Fish oils are a suitable source of eicosapentanoic acids and docosahexanoic acid. Borage oil, blackcurrent seed oil and evening primrose oil are suitable sources of gamma linolenic acid. Safflower oils, sunflower oils, corn oils and soy bean oils are suitable sources of linoleic acid.

If necessary, the pet foods are supplemented with minerals and vitamins so that they are nutritionally complete.

The amount of the pet food to be consumed by the pet to obtain a beneficial effect will depend upon the size or the pet, the type of pet, and age of the pet. However an amount of the pet food to provide a daily amount of about 1g to about 100g of prebiotic, or about 10⁶ to about 10¹² cells of the probiotic micro-organism, would usually be adequate.

Specific examples recording the remarkable effects of feeding an embodiment of the inventive diet according to the invention to elderly pets are now described for further illustration.

### Example 1

A first pet owner in Pretoria, South Africa has two golden retrievers, ages 8 and 9 years. She regarded them as forming an integral part of her family unit and consequently used to provide what she considered the best nutrition available - a conventional senior food from a veterinary brand. Nevertheless she observed that her dogs had poor coat quality and had the decreased activity typical of senior dogs. The owner states that she started feeding her dogs the petfood sold under the brand name Olympic Senior (this is a dry dog food containing an effective amount of inulin prebiotic). She reports having since seen the following changes in her dogs:
- shinier coats,
- a healthy look and,
- a return to being as lively as they had been a few years previously.

"Olympic" is a trade mark belonging to Epol (Proprietary) Limited.

### Example 2

A second pet owner in South Africa reports having an elderly Staffordshire Terrier which was arthritic, moved slowly and was much less active than when younger. In particular, it would not run around. The owner started feeding it a diet of Olympic Senior dry dog food, the same as in Example 1. Within a few months, the dog's activity levels increased, it began again to run around and is reportedly now willing and able to jump over a three foot fence.

### Example 3

A pet owner in Great Britain reported having an elderly Labrador Retriever which was "very arthritic" and had trouble walking up stairs. She began feeding her dog Winalot Complete Digestion+, a dry dog food containing about 1% chicory as a source of the prebiotic, inulin. Within a month on the product, the dog started running around "like a puppy" and "is now bounding up the stairs so fast that he trips over his own feet".

"Winalot" is a trade mark belonging to Societé de Produits Nestlé of Switzerland.

### Example 4

A pet owner in California, USA reports that, after changing his dog's diet to Alpo Complete dry dog food containing about 1% chicory by weight (ALPO is a trade mark of Societé de Produits Nestlé), its coat became noticeably shinier, its eyes brighter and its overall activity levels increased.

A dog owner in Pennsylvania reported similarly that his dog, after changing to a diet of Alpo Complete soon exhibited remarkably improved changes in looks and in "attitude", while a dog owner in West Virginia observed that his dog no long behaved in accordance with its 13 years, but instead seemed far younger.

## Claims

1. A method for improving activity in a pet, the method comprising administering to the pet a nutritional agent which promotes the growth of bifido- and lactic-bacteria in the gastro-intestinal tract of the pet.

2. A method according to claim 1 in which the nutritional agent is a component of a nutritionally complete pet food.

3. A method according to claim 1 in which the nutritional agent is selected from the group of prebiotics and probiotic micro-organisms.

4. A method according to claim 3 in which the prebiotic is selected from the group of inulin, fructooligosaccharides and plant materials which contain inulin and/or fructooligosaccharides.

5. A method according to claim 1 in which the pet food further comprises a long chain fatty acid.

6. A method according to claim 1 in which the pet food further comprises a starch source selected from one or more of corn, rice, barley, and wheat.

7. A method for improving activity in an elderly pet, the method comprising administering to the pet a nutrionally complete pet food which contains a nutritional agent which promotes the growth of bifido- and lactic-bacteria in the gastro-intestinal tract of the pet.

8. A method according to claim 7 in which the nutritional agent is selected from the group of prebiotics and probiotic micro-organisms.

9. A method according to claim 8 in which the prebiotic is selected from the group of inulin, fructooligosaccharides and plant materials which contain inulin and/or fructooligosaccharides.

10. A method according to claim 7 in which the pet food contains about 0.1% to about 5% by weight of a prebiotic as the nutritional agent.

11. A method according to claim 7 in which the pet food contains about 10⁴ to about 10¹⁰ cells of a probiotic micro-organism per gram of the pet food as the nutritional agent.

12. A method according to claim 7 in which the pet food further comprises a long chain fatty acid.

13. A method according to claim 1 in which the pet food further comprises a starch source selected from one or more of corn, rice, barley, and wheat.

14. A method for improving activity in an elderly dog, the method comprising administering to the dog a nutrionally complete pet food which contains a nutritional agent which promotes the growth of bifido- and lactic-bacteria in the gastro-intestinal tract of the dog.

15. Use of a nutritional agent which promotes the growth of bifido- and lactic-bacteria in the gastro-intestinal tract of the pet in the manufacture of a composition for ameliorating joint stiffness in a pet.

16. Use according to claim 15 wherein the nutritional agent assists in improving systemic inflammatory status in the pet.

17. Use according to claim 16 wherein the nutritional agent assists in reducing concentrations of pathogenic bacteria known to contribute to an increased inflammatory status.

18. Use according to any one of claims 15 to 17 in which the pet is an elderly dog.

19. Use according to any one of claims 15 to 17 in which the pet is an elderly cat.

20. A method for improving activity in an elderly dog, the method comprising administering to the dog a nutritionally complete pet food which contains a prebiotic selected from the group of inulin, fructooligosaccharides and plant materials which contain inulin and/or fructooligosaccharides.

21. A method of improving physical activity in an elderly pet comprising administering to the pet a pet food composition comprising about 1% chicory by weight.

22. A method of improving physical activity levels in an elderly pet comprising administering to the pet a pet food composition comprising at least about 1% chicory by weight as a source of inulin.

23. A method of increasing activity level in a dog of at least 5 years of age or a cat of at least 7 years of age, comprising feeding the pet a balanced diet comprising a pet food formulation comprising inulin in an effective amount to reduce joint stiffness.

24. A method of returning a senior pet to being as lively as they had a few years previously comprising administering to the pet a pet food composition comprising inulin in an amount effective to increase physical activity in such pet.

## Patentansprüche

1. Verfahren zur Verbesserung von Aktivität bei einem Haustier, wobei das Verfahren umfasst, Verabreichen eines Nahrungsagens an das Haustier, welches das Wachstum von Bifido- und Milchsäure-Bakterien in dem Gastrointestinaltrakt des Haustiers fordert.

2. Verfahren nach Anspruch 1, wobei das Nahrungsagens eine Komponente eines unter Ernährungsgesichtspunkten vollständigen Haustiernahrungsmittels ist.

3. Verfahren nach Anspruch 1, wobei das Nahrungsagens ausgewählt ist aus der Gruppe von Prebiotika und probiotischen Mikroorganismen.

4. Verfahren nach Anspruch 3, wobei das Prebiotikum ausgewählt ist aus der Gruppe von Inulin, Fructo-Oligosacchariden und Pflanzenmaterialien, die Inulin und/oder Fructo-Oligosaccharide enthalten.

5. Verfahren nach Anspruch 1, wobei das Haustiernahrungsmittel weiter eine langkettige Fettsäure umfasst.

6. Verfahren nach Anspruch 1, wobei das Haustiernahrungsmittel weiter umfasst, eine Stärkequelle, die ausgewählt ist unter einem oder mehreren von Mais, Reis, Gerste und Weizen.

7. Verfahren zur Verbesserung von Aktivität bei einem älteren Haustier, wobei das Verfahren umfasst, Verabreichen eines unter Ernährungsgesichtspunkten vollständigen Haustiernahrungsmittels an das Haustier, das ein Nahrungsagens enthält, welches das Wachstum von Bifido- und Milchsäure-Bakterien in dem Gastrointestinaltrakt des Haustiers fördert.

8. Verfahren nach Anspruch 7, wobei das Nahrungsagens ausgewählt ist aus der Gruppe von Prebiotika und probiotischen Mikroorganismen.

9. Verfahren nach Anspruch 8, wobei das Prebiotikum ausgewählt ist aus der Gruppe von Inulin, Fructo-Oligosacchariden und Pflanzenmaterialien, die Inulin und/oder Fructo-Oligosaccharide enthalten.

10. Verfahren nach Anspruch 7, wobei das Haustiernahrungsmittel etwa 0,1 % bis etwa 5 Gew.-% eines Prebiotikums als das Nahrungsagens enthält.

11. Verfahren nach Anspruch 7, wobei das Haustiernahrungsmittel etwa 10⁴ bis etwa 10¹⁰ Zellen eines probiotischen Mikroorganismus pro Gramm des Haustiernahrungsmittels als das Nahrungsagens enthält.

12. Verfahren nach Anspruch 7, wobei das Haustiernahrungsmittel weiter eine langkettige Fettsäure umfasst.

13. Verfahren nach Anspruch 1, wobei das Haustiernahrungsmittel weiter umfasst, eine Stärkequelle, die ausgewählt ist unter einem oder mehreren von Mais, Reis, Gerste und Weizen.

14. Verfahren zur Verbesserung von Aktivität bei einem älterem Hund, wobei das Verfahren umfasst, Verabreichen eines unter Ernährungsgesichtspunkten vollständigen Haustiernahrungsmittels an den Hund, das ein Nahrungsagens enthält, welches das Wachstum von Bifido- und Milchsäure-Bakterien in dem Gastrointestinaltrakt des Hundes fördert.

15. Verwendung eines Nahrungsagens, welches das Wachstum von Bifido- und Milchsäure-Bakterien in dem Gastrointestinaltrakt des Haustiers fordert, zur Herstellung einer Zusammensetzung zum Verbessern von Gelenksteifheit bei einem Haustier.

16. Verwendung nach Anspruch 15, wobei das Nahrungsagens beim Verbessern eines systemischen Entzündungsstatus in dem Haustier hilft.

17. Verwendung nach Anspruch 16, wobei das Nahrungsagens bei einem Verringern von Konzentrationen an pathogenen Bakterien hilft, von denen bekannt ist, dass sie zu einem erhöhtem Entzündungsstatus beitragen.

18. Verwendung nach einem der Ansprüche 15 bis 17, wobei das Haustier ein älterer Hund ist.

19. Verwendung nach einem der Ansprüche 15 bis 17, wobei das Haustier eine ältere Katze ist.

20. Verfahren zur Verbesserung von Aktivität bei einem älteren Hund, wobei das Verfahren umfasst, Verabreichen eines unter Ernährungsgesichtspunkten vollständigen Haustiernahrungsmittels an den Hund, welches ein Prebiotikum enthält, das ausgewählt ist aus der Gruppe von Inulin, Fructo-Oligosacchariden und Pflanzenmaterialien, die Inulin und/oder Fructo-Oligosaccharide enthalten.

21. Verfahren zur Verbesserung von körperlicher Aktivität bei einem älteren Haustier, welches umfasst, Verabreichen einer Haustiernahrungsmittelzusammensetzung an das Haustier, die etwa 1 Gew.-% an Chicorée umfasst.

22. Verfahren zur Verbesserung körperlicher Aktivitätsgrade bei einem älteren Haustier, welches umfasst, Verabreichen einer Haustiernahrungsmittelzusammensetzung an das Haustier, welche umfasst, mindestens etwa 1 Gew.-% an Chicorée als eine Inulinquelle.

23. Verfahren zum Erhöhen eines Aktivitätsgrads in einem Hund mit einem Alter von mindestens 5 Jahren oder einer Katze mit einem Alter von mindestens 7 Jahren, welches umfasst, Füttern des Haustiers mit einer ausgewogenen Diät, welche umfasst, eine Haustiernahrungsmittelformulierung, die Inulin in einer wirksamen Menge zum Verringern von Gelenksteifheit umfasst.

24. Verfahren zum Zurückbringen einer Lebhaftigkeit eines älteren Haustiers, wie sie bei diesem einige Jahre zuvor war, welches umfasst, Verabreichen einer Haustiernahrungsmittelzusammensetzung an das Haustier, welche Inulin in einer wirksamen Menge zum Steigern von körperlicher Aktivität in einem derartigen Haustier umfasst.

## Revendications

1. Procédé pour améliorer l'activité chez un animal familier, le procédé comprenant l'administration à l'animal d'un agent nutritionnel qui favorise la croissance des bifidobactéries et des bactéries lactiques dans le tractus gastro-intestinal de l'animal.

2. Procédé selon la revendication 1, dans lequel l'agent nutritionnel est un composant d'un aliment nutritionnellement complet pour animaux familiers.

3. Procédé selon la revendication 1, dans lequel l'agent nutritionnel est choisi dans le groupe des prébiotiques et des micro-organismes probiotiques.

4. Procédé selon la revendication 3, dans lequel le prébiotique est choisi dans le groupe de l'inuline, des fructo-oligosaccharides et des matières végétales qui contiennent de l'inuline et/ou des fructo-oligosaccharides.

5. Procédé selon la revendication 1, dans lequel l'aliment pour animaux familiers comprend de plus un acide gras à longue chaîne.

6. Procédé selon la revendication 1, dans lequel l'aliment pour animaux familiers comprend de plus une source d'amidon choisie parmi l'un ou plusieurs du maïs, du riz, de l'orge et du blé.

7. Procédé pour améliorer l'activité chez un animal familier âgé, le procédé comprenant l'administration à l'animal d'un aliment nutritionnellement complet pour animaux familiers qui contient un agent nutritionnel qui favorise la croissance des bifidobactéries et des bactéries lactiques dans le tractus gastro-intestinal de l'animal.

8. Procédé selon la revendication 7, dans lequel l'agent nutritionnel est choisi dans le groupe formé par les prébiotiques et les microorganismes probiotiques.

9. Procédé selon la revendication 8, dans lequel le prébiotique est choisi dans le groupe de l'inuline, des fructo-oligosaccharides et des matières végétales qui contiennent de l'inuline et/ou des fructo-oligosaccharides.

10. Procédé selon la revendication 7, dans lequel l'aliment pour animaux familiers contient environ 0,1 % à environ 5 % en poids d'un prébiotique comme agent nutritionnel.

11. Procédé selon la revendication 7, dans lequel l'aliment pour animaux familiers contient environ 10⁴ à environ 10¹⁰ cellules d'un micro-organisme probiotique par gramme de l'aliment pour animaux familiers à titre de l'agent nutritionnel.

12. Procédé selon la revendication 7, dans lequel l'aliment pour animaux familiers comprend de plus un acide gras à longue chaîne.

13. Procédé selon la revendication 1, dans lequel l'aliment pour animaux familiers comprend de plus une source d'amidon choisie parmi l'un ou plusieurs du maïs, du riz, de l'orge et du blé.

14. Procédé pour améliorer l'activité chez un chien âgé, le procédé comprenant l'administration au chien d'un aliment nutritionnellement complet pour animaux familiers qui contient un agent nutritionnel qui favorise la croissance des bifidobactéries et des bactéries lactiques dans le tractus gastro-intestinal du chien.

15. Utilisation d'un agent nutritionnel qui favorise la croissance des bifidobactéries et des bactéries lactiques dans le tractus gastro-intestinal de l'animal familier dans la fabrication d'une composition pour atténuer la raideur articulaire chez un animal familier.

16. Utilisation selon la revendication 15, dans laquelle l'agent nutritionnel aide à améliorer l'état inflammatoire général chez l'animal familier.

17. Utilisation selon la revendication 16, dans laquelle l'agent nutritionnel aide à réduire les concentrations de bactéries pathogènes connues pour contribuer à un état inflammatoire accru.

18. Utilisation selon l'une quelconque des revendications 15 à 17, dans laquelle l'animal familier est un chien âgé.

19. Utilisation selon l'une quelconque des revendications 15 à 17, dans laquelle l'animal familier est un chat âgé.

20. Procédé pour améliorer l'activité chez un chien âgé, le procédé comprenant l'administration au chien d'un aliment nutritionnellement complet pour animaux familiers qui contient un prébiotique choisi dans le groupe de l'inuline, des fructo-oligosaccharides et des matières végétales qui contiennent de l'inuline et/ou des fructo-oligosaccharides.

21. Procédé pour améliorer l'activité physique chez un animal familier âgé, comprenant l'administration à l'animal d'une composition d'aliment pour animaux familiers comprenant environ 1 % en poids de chicorée.

22. Procédé pour améliorer les niveaux d'activité physique chez un animal familier âgé, comprenant l'administration à l'animal d'une composition d'aliment pour animaux familiers comprenant au moins environ 1 % en poids de chicorée comme source d'inuline.

23. Procédé pour augmenter le niveau d'activité chez un chien ayant un âge d'au moins 5 ans ou un chat ayant un âge d'au moins 7 ans, comprenant l'alimentation de l'animal avec un régime équilibré comprenant une formulation d'aliment pour animaux familiers comprenant de l'inuline en une quantité efficace pour réduire la raideur articulaire.

24. Procédé pour rendre un animal familier aussi vif que lorsqu'il avait quelques années de moins, comprenant l'administration à l'animal d'une composition d'aliment pour animaux familiers comprenant de l'inuline en une quantité efficace pour augmenter l'activité physique chez cet animal.
